# EUROPEAN PATENT APPLICATION

(11) **EP 2 543 354 A1**
(43) Date of publication of application: **09.01.2013**
(21) Application number: 11750684.0
(22) Date of filing: 02.03.2011
(51) Int. Cl.: A61J 3/00

(54) **MEDICAL DEVICE**

(30) Priority: 05.03.2010 JP 2010049760
(71) Applicant: Terumo Kabushiki Kaisha, Tokyo 151-0072 (JP)
(72) Inventor: TAKEMOTO Masafumi, Nakakoma-gun Yamanashi 409-3853 (JP); KOYAMA Shingo, Nakakoma-gun Yamanashi 409-3853 (JP)
(74) Representative: TBK
(86) International application number: PCT/JP2011/054723
(87) International publication number: WO 2011/108575

(57) **Abstract**

A double-ended needle assembly, as a medical device, is provided with: a double-ended needle having a tip-end needle tip and a base-end needle tip; a support member which is attached to the spout of a syringe, seals the spout in a liquid tight manner while being attached thereto, has a needle insertion section that allows the base-end needle tip to be inserted, and supports the double-ended needle in a manner such that the double-ended needle can move along the axial direction thereof; and a cover member which is secured in relation to the double-ended needle, covers the tip-end needle tip while being secured, and moves the double-ended needle. In the initial state, the tip-end needle tip is covered by the cover member and the base-end needle tip is not inserted into the needle insertion section. When the state is shifted from the initial state to a state in which the cover member moves in the base end direction along with the double-ended needle, the base-end needle tip of the double-ended needle is inserted into the needle insertion section. Then, the secured state of the cover member in relation to the double-ended needle is released, and the cover member can separate from the double-ended needle.

## Description

### Technical Field

The present invention relates to a medical device.

### Background Art

There has heretofore been known a prefilled syringe previously filled with liquid such as a heparin solution or the like (see, for example, Patent Document 1). The prefilled syringe described in Patent Document 1 includes a syringe outer tube having a spout through which the liquid is discharged and a cap which liquid-tightly seals the spout of the syringe outer tube. When the prefilled syringe is used, the cap is first removed from the spout of the syringe outer tube. Then, for example, an injection needle is connected to the spout from which the cap has been removed. Thus, the prefilled syringe can be used.

However, in the prefilled syringe described in Patent Document 1, the cap is simply fitted to and thus attached to the spout of the syringe outer tube. Thus, if the cap is only pulled in the distal end direction, the cap will easily be detached from the spout. Therefore, although the prefilled syringe is not intended to be used, if a certain amount of force is applied to the cap in the distal end direction, the cap is detached involuntarily thereby to bring the prefilled syringe involuntarily into a usable state, adversely. The prefilled syringe in this state has to be used at once or to be discarded.
Patent Document 1: Japanese Laid-Open Patent Publication No. 2007-084579

### Summary of Invention

It is an object of the present invention to provide a medical device that can surely prevent a cover member covering a double-ended needle from being involuntarily detached from the double-ended needle.

To achieve the above object, the present invention is a medical device including a liquid injection device having at a distal end portion a spout through which liquid passes and a double-ended needle assembly attached to the spout of the liquid injection device,
the double-ended needle assembly including:
a double-ended needle including a needle tube having incisive needle tips at both ends, and a hub for supporting the needle tube;
a support member attached to the spout and which include a puncture portion for liquid-tightly sealing the spout in the attached state and being capable of being punctured by the needle tip on a proximal end side of the needle tube, the support member supporting the double-ended needle movably in axial directions of the double-ended needle; and
a cover member including a fixing portion fixed to the double-ended needle, the cover member covering the needle tip on a distal end side of the needle tube in a state where the fixing portion is fixed, and being operated to displace the double-ended needle along the axial directions of the double-ended needle;
wherein in an initial state, the needle tip on the distal end side of the needle tube of the double-ended needle is covered by the cover member and the needle tip on the proximal end side of the needle tube thereof is in a not-yet-punctured state with respect to the puncture portion, and
during a period of time from the initial state to a usable state where the cover member, along with the double-ended needle, is displaced in the proximal end direction, in the double-ended needle, the needle tip on the proximal end side of the needle tube punctures the puncture portion so that the needle tube communicates with the liquid injection device, and fixation of the fixing portion to the double-ended needle is released so that it becomes possible for the cover member to be detached from the double-ended needle.

Preferably, in the medical device of the present invention, the communication between the needle tube and the liquid injection device is done at the same timing as or at an earlier timing than the release of the fixation of the cover member to the double-ended needle.

Preferably, in the medical device of the present invention, the hub is composed of a tubular body,
the cover member is composed of a tubular body disposed on the outer side of the hub, and has a fixing projection for functioning as the fixing portion, the fixing projection being formed on an inner circumferential portion of the cover member so as to project therefrom, and
the fixing projection engages with a side wall of the hub in the initial state and thus is fixed to the double-ended needle, and in the usable state the fixing projection is pushed outwardly by a portion of the liquid injection device and is separated from the side wall of the hub, whereby the engagement of the fixing projection with the side wall of the hub is released.

Preferably, in the medical device of the present invention, the side wall of the cover member is composed of a plurality of small pieces that are supported at respective distal ends in a cantilever manner and disposed along a circumferential direction thereof, and connecting portions for connecting proximal end portions of the small pieces that are adjacent to each other, and the fixing projection is disposed on the connecting portion.

Preferably, the medical device of the present invention further includes positioning means for axially positioning the double-ended needle with respect to the support member in each of the initial state and the usable state.

Preferably, in the medical device of the present invention, the hub is composed of a tubular body,
the support member is composed of a tubular body disposed on the inner side of the hub, and
the positioning means includes a positioning projection projecting from a side wall of one of the hub and the support member; a first engaging portion provided on a side wall of the other of the hub and the support member and which engages with the positioning projection in the initial state; and a second engaging portion provided closer to a proximal end side than the first engaging portion and which engages with the positioning projection in the usable state.

Preferably, in the medical device of the present invention, a distance until the needle tip on the proximal end side of the needle tube punctures the puncture portion is the same as or shorter than a distance until the fixation of the fixing portion is released.

Preferably, in the medical device of the present invention, the fixing projection has a proximal end portion inclined toward the inside.

Preferably, in the medical device of the present invention, the other side wall is formed thereon with a slanted portion between the first engaging portion and the second engaging portion, the inclination angle of the slanted portion being increased toward a proximal end direction.

Preferably, in the medical device of the present invention, the puncture portion is composed of a plate-like elastic body.

Preferably, in the medical device of the present invention, the liquid injection device is a syringe.

### Brief Description of Drawings

FIG. 1 is a longitudinal cross-sectional view sequentially showing an operational process of a medical device of the present invention;
FIG. 2 is a longitudinal cross-sectional view sequentially showing an operational process of the medical device of the present invention;
FIG. 3 is a longitudinal cross-sectional view sequentially showing an operational process of the medical device of the present invention;
FIG. 4 is a longitudinal cross-sectional view sequentially showing an operational process of the medical device of the present invention;
FIG. 5 is a perspective view showing a cover member and a double-ended needle included in the medical device of the present invention; and
FIG. 6 is a longitudinal cross-sectional perspective view of the cover member and the double-ended needle shown in FIG. 5.

### Description of Embodiments

A medical device of the present invention will hereinafter be described in detail with reference to preferred embodiments shown in the accompanying drawings.

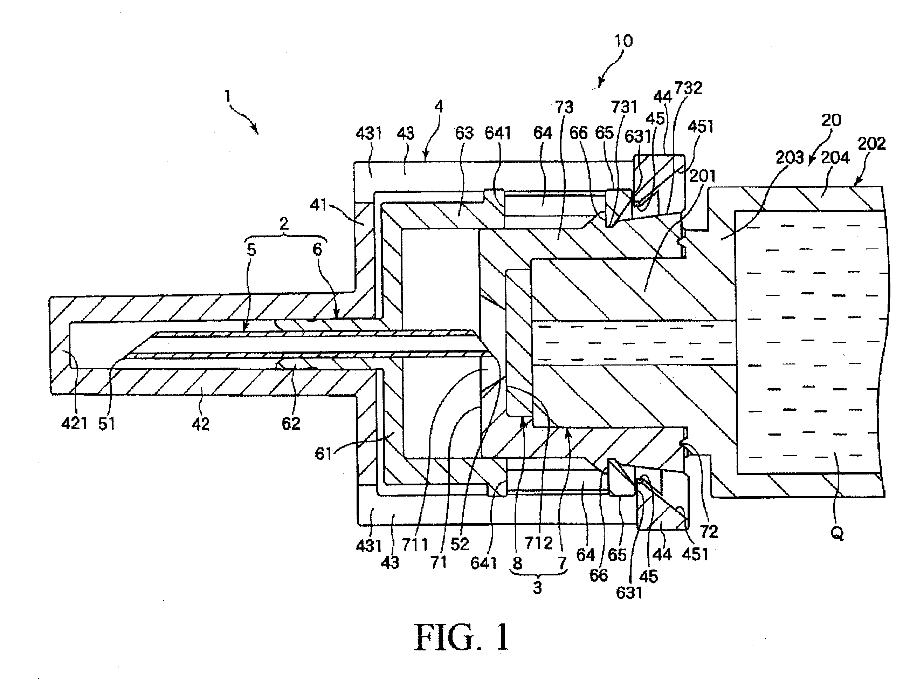

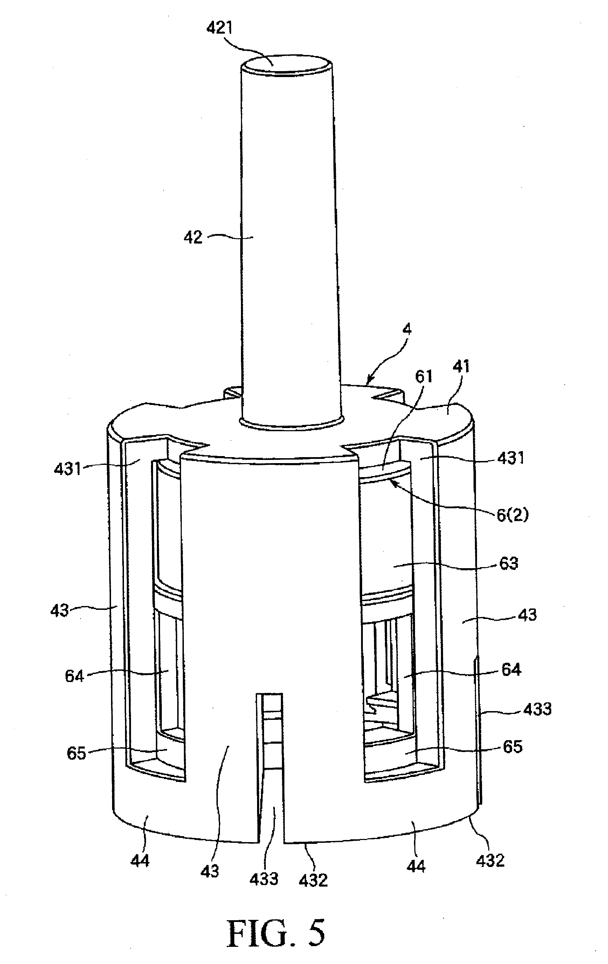

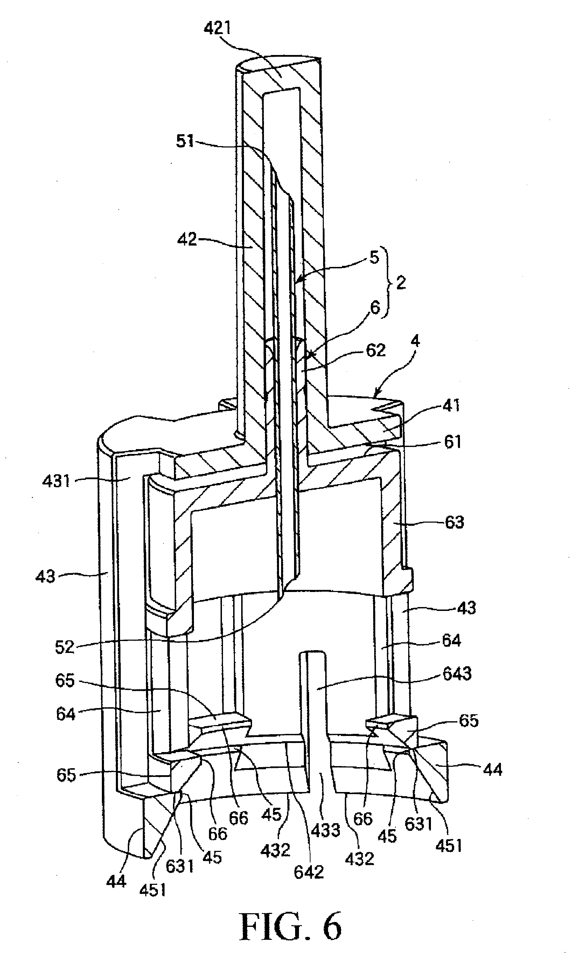

FIGS. 1 to 4 are longitudinal cross-sectional views sequentially showing the operational processes of the medical device of the present invention. FIG. 5 is a perspective view showing a cover member and a double-ended needle included in the medical device of the present invention. FIG. 6 is a longitudinal cross-sectional perspective view of the cover member and the double-ended needle shown in FIG. 5. Incidentally, for the convenience of the following description, the right side in FIGS. 1 to 4 is referred to as "the proximal end" and the left side is referred to as "the distal end." In FIGS. 5 and 6, the downside is referred to as "the proximal end" and the upside is referred to as "the distal end."

A medical device 10 shown in FIGS. 1 to 4 includes a prefilled syringe (hereinafter, simply called "the syringe") 20 which is a liquid injection device and a double-ended needle assembly 1 attached to a spout 201 of the syringe 20. The constitution of each part is described below.

The syringe 20 has a syringe outer tube 202 having a bottomed tubular shape. Liquid Q is previously filled in the syringe outer tube 202.

A bottom portion (a distal end portion) 203 of the syringe outer tube 202 has a tubular spout 201 formed projectingly toward a distal end direction. The liquid Q can pass through the spout 201.

The liquid Q is not particularly limited. Various drag solutions can be used as the liquid Q.

The double-ended needle assembly 1 shown in FIGS. 1 to 3 includes a double-ended needle 2, a support member 3 supporting the double-ended needle 2, and a cover member 4 covering the double-ended needle 2. The double-ended needle assembly 1 is assembled by stacking the cover member 4, the double-ended needle 2 and the support member 3 in this order from the distal end side (see FIG. 1).

The double-ended needle 2 has a needle tube 5 and a hub 6 supporting a longitudinal middle portion of the needle tube 5.

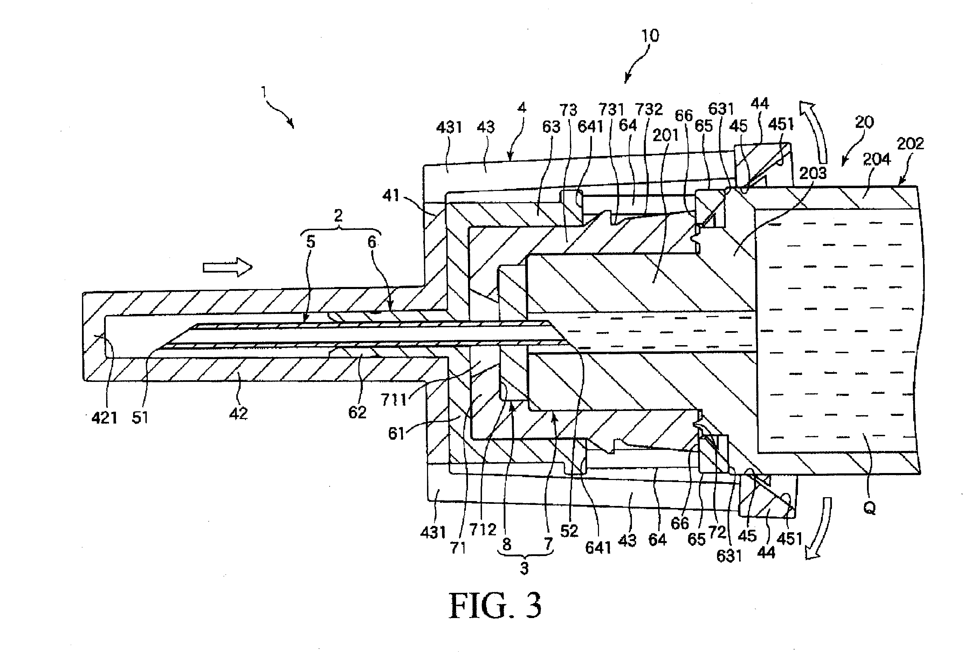

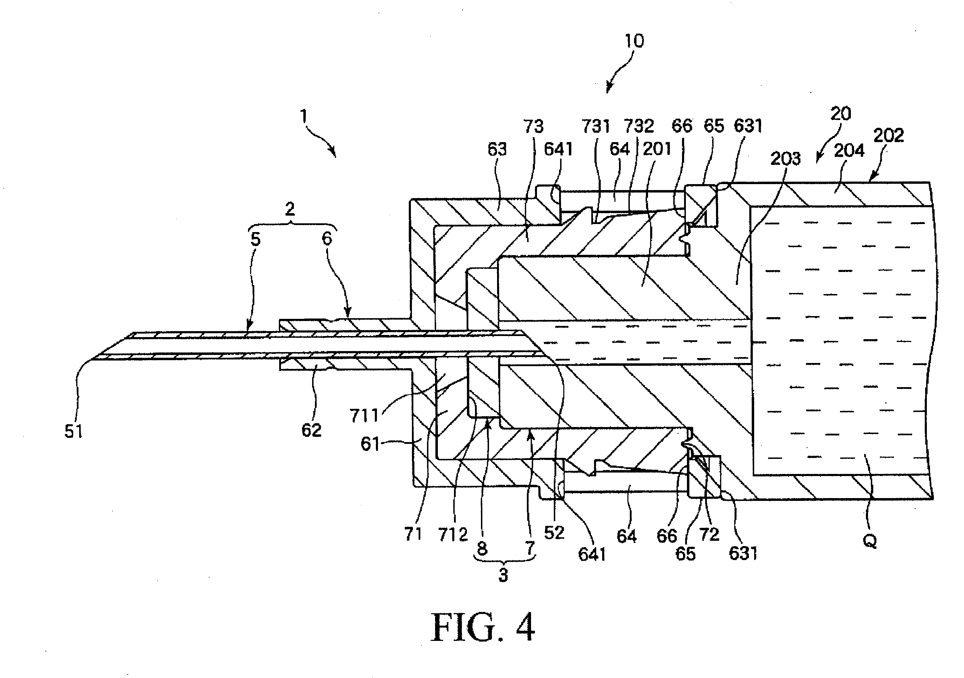

The needle tube 5 has an incisive distal end needle tip (a distal end side needle tip) 51 at the distal end and an incisive proximal end needle tip (a proximal end side needle tip) 52 at the proximal end. In the double-ended needle assembly 1, in an initial state before use shown in FIG. 1, the distal end needle tip 51 is covered by the cover member 4, whereas the proximal end needle tip 52 is in a not-yet-punctured state with respect to a puncture portion 8 of the support member 3 to be described later. In a usable state shown in FIGS. 3 and 4, the cover member 4 is removed to expose the distal end needle tip 51 and the proximal end needle tip 52 punctures the puncture portion 8 of the support member 3.

Incidentally, although the constituent material of the needle tube 5 is not particularly limited, examples of the constituent material include various metal materials such as stainless steel.

As shown in FIG. 1, the hub 6 is composed of a bottomed tubular member. A bottom portion 61 of the hub 6 is formed with a tubular support portion 62 projecting toward the distal end direction. The needle tube 5 is inserted through and fitted in the support portion 62. Thus, the needle tube 5 can be supported and secured.

As shown in FIGS. 5 and 6, a side wall 63 of the hub 6 has a proximal end side portion composed of a plurality of small pieces 64 (four pieces in the present embodiment) and connecting portions 65 each connecting the proximal end portions of the small pieces 64 that are adjacent to each other.

The small pieces 64 are arranged at equal angular intervals around the axis of the hub 6. Each of the small pieces 64 is supported at a distal end 641 thereof in a cantilever manner by the distal end side portion of the side wall 63. Further, the small pieces 64 are each formed with a slit 643 extending from the proximal end 642 to a middle portion thereof. As a result, each of the small pieces 64 can elastically deform with the distal end 641 serving as a fixed end (see FIGS. 1 to 4). Incidentally, the small pieces 64 are each formed with the slit 643 in the illustrated constitution; however, the present invention is not limited to this constitution. The slit 643 may be omitted. In this case, the connecting portion 65 can be omitted. Alternatively, small pieces 64 connected to a connecting portion 65 and small pieces 64 not connected to a connecting portion 65 may alternately be arranged around the axis of the hub 6.

The connecting portions 65 are each formed into an arch shape and a positioning projection 66 is formed at the internal circumferential portion thereof so as to project therefrom. The positioning projection 66 is a portion for functioning as part of positioning means to be described later. The positioning projection 66 can be displaced in the vertical directions in FIGS. 1 to 4 by elastic deformation of the corresponding small piece 64.

Incidentally, although the constituent material of the hub 6 is not particularly limited, examples of the constituent material include various resins such as polyethylene and polypropylene.

As shown in FIGS. 1 to 4, the double-ended needle 2 is supported by the support member 3. The support member 3 can support the double-ended needle 2 such that the double-ended needle 2 can be displaced along the axial direction thereof and the support member 3 is attached to the spout 201 of the syringe 20.

The support member 3 has a main body 7 formed into a bottomed tubular shape and the puncture portion 8 mounted at a bottom portion 71 of the main body 7.

The main body 7 is concentrically disposed inside the hub 6 of the double-ended needle 2 and attached to the spout 201 of the syringe 20. Incidentally, it is preferred that the main body 7 be secured to the spout 201 of the syringe 20. This securing method is not particularly limited. Examples of the securing method include fitting-into, adhesion (adhesion using an adhesive or solvent) and fusion (thermal fusion bonding, high-frequency fusion, ultrasonic fusion). In this case, it is preferred that a securing position of the main body 7 to the syringe 20 be at the proximal end 72 of the main body 7.

As shown in FIG. 1, the side wall 73 of the main body 7 is formed with a recessed portion 731 for functioning as a first engaging portion. Each of the positioning projections 66 of the hub 6 of the double-ended needle 2 engages with the first engaging portion in the initial state. The recessed portion 731 is formed into a ring shape extending along the circumferential direction of the side wall 73 of the main body 7. In this way, the positioning projections 66 can collectively engage with the recessed portion 731. Owing to this engagement, the double-ended needle 2 is axially positioned with respect to the support member 3 in the initial state. Thus, the involuntary displacement of the double-ended needle can be prevented.

As shown in FIG. 3, in the usable state, the positioning projections 66 of the double-ended needle 2 engage with the proximal end 72 which is a portion located closer to the proximal end than the recessed portion 731 of the main body 7. The proximal end 72 functions as a second engaging portion for engaging with the positioning projections 66 in the usable state. The positioning projections 66 engage with the proximal end 72 of the main body 7, so that the double-ended needle 2 is axially positioned with respect to the support member 3 in the usable state. Thus, involuntary displacement of the double-ended needle 2 can be prevented.

As described above, in the double-ended needle assembly 1, the positioning projections 66 of the double-ended needle 2 and the recessed portion 731 and proximal end 72 of the main body 7 jointly forms a positioning means for axially positioning the double-ended needle 2 with respect to the support member 3 in each of the initial state and the usable state.

Incidentally, the second engaging portion of the positioning means is the proximal end 72 of the main body 7 in the constitution shown in FIG. 3. However, the present invention is not limited to this respect. For example, the second engaging portion may be a recessed portion formed between the recessed portion 731, which functions as the first engaging portion, and the proximal end 72, of the side wall 73 of the main body 7.

A slanted portion 732 is formed on a portion of the side wall 73 of the main body 7 between the recessed portion 731 and the proximal end 72. The inclination angle of the slanted portion 732 is increased as it goes toward the proximal end direction. Owing thereto, it is possible to surely prevent release of a state where the positioning projections 66 of the double-ended needle 2 engage with the proximal end 72 of the main body 7 thereby to position the double-ended needle 2 in the usable state. In other words, it is possible to surely prevent the double-ended needle 2 having been displaced in the proximal end direction from returning again in the distal end direction.

The bottom portion 71 of the main body 7 is formed thereon with a through-hole 711. The through-hole 711 has a proximal end side portion which increases in inner diameter. The proximal end side portion serves as a puncture-portion mounting portion 712 in which the puncture portion 8 is mounted.

Incidentally, the constituent material of the main body 7 is not particularly limited. For example, the constituent material of the hub 6 described above can be used as that of the main body 7. In this case, the constituent material of the main body 7 may be the same as or different from that of the hub 6.

The puncture portion 8 mounted in the puncture-portion mounting portion 712 can liquid-tightly seal the spout 201 of the syringe 20 in the initial state (i.e., in the attached state) shown in FIG. 1. Thus, the liquid Q can surely be prevented from leaking involuntarily from the spout 201.

The puncture portion 8 is punctured by the proximal end needle tip 52 of the double-ended needle 2 before the usable state shown in FIG. 3 is achieved. In this way, the needle tube 5 of the double-ended needle 2 communicates with the syringe outer tube 202 of the syringe 20 and thus, the liquid Q can be discharged via the needle tube 5.

The puncture portion 8 as described above is composed of a disk-like elastic body. With this, the puncture portion 8 can surely seal the spout 201 of the syringe 20 in the initial state. In addition, the puncture portion 8 can be punctured easily and reliably by the proximal end needle tip 52 of the double-ended needle 2 in the usable state.

Incidentally, the elastic material forming the puncture portion 8 is not particularly limited. Examples of the elastic material include various rubber materials such as natural rubber, butyl rubber, isoprene rubber, butadiene rubber, styrene-butadiene rubber and silicone rubber; polyurethane-based, polyester-based, polyamide-based, olefin-based, styrene-based or other-based thermoplastic elastomer; and mixtures thereof.

As described above, the double-ended needle 2 is supported by the support member 3 in such a manner as to be able to be displaced along the axial direction of thereof. The displacement of the double-ended needle 2 can be performed by gripping and operating the cover member 4 attached to the outer side of the double-ended needle 2.

The cover member 4 is composed of a bottomed tubular member.

The cover member 4 has a bottom portion 41 formed with a tubular portion 42 formed into a tubular shape projecting in the distal end direction. The tubular portion 42 is closed at its distal end 421, which can cover the distal end needle tip 51 of the needle tube 5 of the double-ended needle 2 (see FIGS. 1 and 6). In this way, the distal end needle tip 51 can be prevented from being exposed, thereby surely preventing erroneous puncture by the distal end needle tip 51.

As shown in FIGS. 5 and 6, the side wall of the cover member 4 is composed of a plurality of (four in the present embodiment) small pieces 43 and connecting portions 44 each connecting the proximal end portions of the small pieces 43 that are adjacent to each other.

The small pieces 43 are arranged at equal angular intervals around the axis of the cover member 4. The small pieces 43 are each supported at its distal end 431 in a cantilever manner by the bottom portion 41 of the cover member 4. The small pieces 43 are each formed therein with a slit 433 extending from its proximal end 432 to a middle portion thereof. In this way, the small pieces 43 can each elastically deform with its distal end 431 serving as a fixed end (see FIGS. 1 to 3). Incidentally, the small pieces 43 are each formed with the slit 433 in the illustrated constitution. However, the present invention is not limited to this respect. The slit 433 may be omitted. In this case, the connecting portion 44 can be omitted. Alternatively, small pieces 43 connected to a connecting portion 44 and small pieces 43 not connected to a connecting portion 44 may be arranged alternately around the axis of the cover member 4.

The connecting portions 44 are each formed into an arch shape, and a fixing projection 45 is formed at the internal circumferential portion thereof so as to project therefrom. The fixing projection 45 functions as a fixing portion fixed to the double-ended needle 2. The fixing projection 45 can be displaced vertically in FIGS. 1 to 3 by elastic deformation of the corresponding small piece 43.

As shown in FIG. 1, the fixing projections 45 can be engaged with a proximal end 631 of the side wall 63 of the hub 6 of the double-ended needle 2 in the initial state. Owing thereto, the cover member 4 is secured to the double-ended needle 2, so that the cover member 4 can surely be prevented from being detached from the double-ended needle 2. In this state, the cover member 4 along with the double-ended needle 2 can be operated to be displaced along the axial direction thereof.

As shown in FIG. 3, the cover member 4 is operated in the proximal end direction thereby to change from the initial state to the usable state. In the usable state, the fixing projections 45 are each pressed outwardly toward an outer circumferential portion 204 of the syringe 20 and are each separated from the proximal end 631 of the hub 6 of the double-ended needle 2. In this way, the engagement of the fixing projections 45 with the proximal end 631 of the hub 6 is released, whereby the cover member 4 can be detached from the double-ended needle 2.

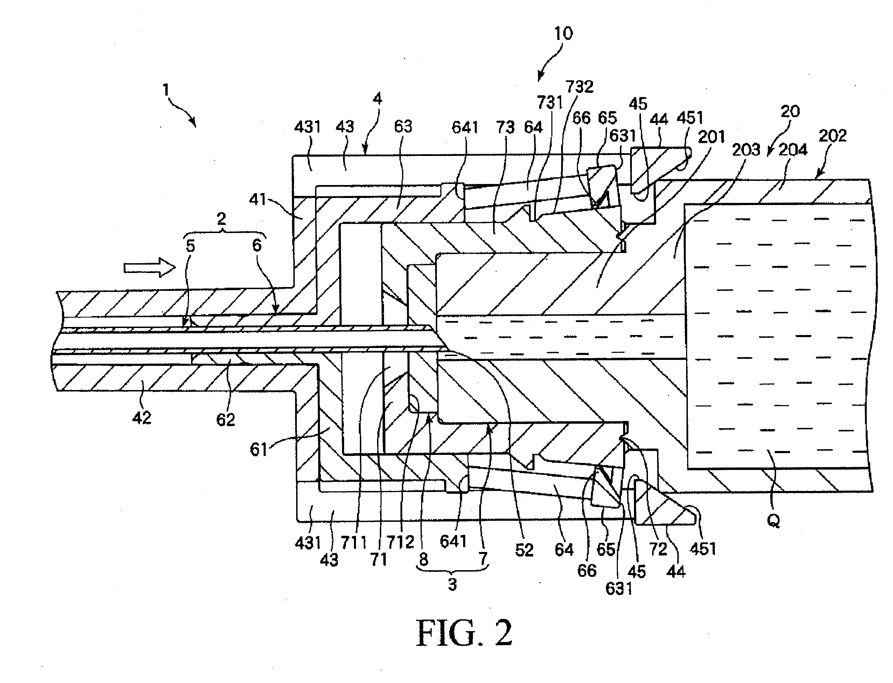

The fixing projections 45 have respective proximal end portions each formed with a slanted portion 451. The slanted portion 451 is a portion where the height of the fixing projection 45 is gradually reduced toward the proximal end direction, i.e., is slanted inwardly. When the outer circumferential portion 204 of the syringe 20 presses the fixing projections 45 as describe above, the outer circumferential portion 204 can push away the slanted portions 451 (see FIG. 2). Thus, such pressing can be performed easily.

As shown in FIG. 6 (also as shown in FIGS. 1 to 3), it is preferred that the fixing projections 45 be each disposed at the same position as a corresponding one of the positioning projections 66 of the double-ended needle 2 around the axis of the double-ended needle 2.

The constituent material of the cover member 4 is not particularly limited. For example, the constituent material of the hub 6 described above can be used as that of the cover member 4. In this case, the constituent material of the cover member 4 may be the same as or different from that of the hub 6.

Next, an operational sequence for operating the double-ended needle assembly 1 and operational states of the double-ended needle assembly 1 will be described with reference to FIGS. 1 to 4.

[1] As shown in FIG. 1, the double-ended needle assembly 1 is previously attached to the spout 201 of the syringe 20.
As described above, in the initial state, the cover member 4 is secured to the double-ended needle 2 and covers the distal end needle tip 51 of the needle tube 5. In the initial state, the positioning projections 66 of the hub 6 are each engaged with the recessed portion 731 of the support member 3, whereby the double-ended needle 2 is positioned. In this state, the double-ended needle 2 is in a not-yet-punctured state where the proximal end needle tip 52 of the needle tube 5 does not yet puncture the puncture portion 8 of the support member 3. Thus, the not-yet-punctured state can surely be maintained until the cover member 4 is operated.

[2] Next, from the state shown in FIG. 1, the cover member 4 is gripped and displaced in the proximal end direction. Then, the cover member 4 along with the double-ended needle 2 is displaced in the proximal end direction (see FIG. 2). In this case, the positioning projections 66 of the double-ended needle 2 are detached from the recessed portion 731 of the support member 3 and climb the slanted portion 732.
As shown in FIG. 2, in the middle of the displacement, the proximal end needle tip 52 of the needle tube 5 of the double-ended needle 2 is puncturing (or starts to puncture) the puncture portion 8 of the support member 3. However, the fixation of the cover member 4 to the double-ended needle 2 is not yet released. Thus, unless the needle tube 5 of the double-ended needle 2 punctures the puncture portion 8 through (i.e., a state shown in FIG. 3), the cover member 4 is prevented from being detached from the double-ended needle 2 even if operation of moving the cover member 4 toward the proximal end direction is stopped halfway and operation of moving the cover member 4 back toward the distal end direction is performed. As described above, in the double-ended needle assembly 1, the needle tube 5 of the double-ended needle 2 punctures the puncture portion 8 thereby to communicate the needle tube 5 with the syringe 20 at an earlier timing than (or at the same timing as) the release of the fixation of the cover member 4 to the double-ended needle 2. Incidentally, the hole formed in the puncture portion 8 by the puncture of the needle tube 5 has self-closing ability because the puncture portion 8 is made of elastic material. In this way, even in the case where the cover member 4 is operated to be returned in the proximal end direction, if the needle tube 5 is removed from the puncture portion 8 by such operating, the hole is closed. Thus, the liquid-tightness of the spout 201 of the syringe 20 is maintained.

[3] From the state shown in FIG. 2, the cover member 4 is further displaced in the proximal end direction, thereby to change to the usable state (see FIG. 3). In the usable state, each of the fixing projections 45 of the cover member 4 climbs the outer circumferential portion 204 of the syringe 20 and is outwardly pushed by the circumferential portion 204. In this way, the fixing projections 45 are each separated from and detached from the proximal end 631 of the hub 6 of the double-ended needle 2. Then, the fixation of the cover member 4 to the double-ended needle 2 is released. With this release, the cover member 4 can be detached from the double-ended needle 2.
In the double-ended needle 2, the positioning projections 66 of the hub 6 climb over the slanted portion 732 of the support member 3 and engage with the proximal end 72. In this way, the double-ended needle 2 is positioned. Thus, when the cover member 4 is operated to be detached, the double-ended needle 2, along with the cover member 4, is pulled in the distal end direction, so that the double-ended needle 2 can surely be prevented from being detached from the syringe 20.
As described above, in the usable state, the needle tube 5 of the double-ended needle 2 communicates with the syringe 20.

[4] Next, as shown in FIG. 4, the double-ended needle assembly 1 is brought into the state where the cover member 4 is removed. In addition, a plunger (not shown) of the syringe 20 is operated to inject the liquid Q.
In the double-ended needle assembly 1 constituted as described above, the cover member 4 covering the double-ended needle 2 can surely be prevented from being involuntarily detached from the double-ended needle 2. In other words, the cover member 4 can surely be prevented from being detached from the double-ended needle 2 before the needle tube 5 of the double-ended needle 2 punctures the puncture portion 8 to communicate with the syringe 20.
The medical device of the present invention has been described above with reference to the illustrated embodiment. However, the present invention is not limited to this embodiment. The portions constituting the medical device can each be replaced with any member that can exhibit the same function. Alternatively, any member may be added thereto.
The present embodiment uses the syringe as the liquid injection device by way of example. However, the present invention is not limited to this. Examples of the liquid injection device include an infusion bag, and a connector attached to one end of a tube.
The positioning means of the present embodiment is constituted such that the positioning projection is provided on the hub of the double-ended needle and the first and second engaging portions are provided on the main body of the support member. However, the present invention is not limited to these constitutions. The positioning means may be constituted such that the positioning projections are provided on the main body of the support member and the first and second engaging portions are provided on the hub of the double-ended needle.

### Industrial Applicability

The medical device of the present invention includes a liquid injection device having at a distal end portion a
spout through which liquid passes and a double-ended needle assembly attached to the spout of the liquid injection device. The double-ended needle assembly includes: a double-ended needle including a needle tube having incisive needle tips at both ends, and a hub for supporting the needle tube; a support member attached to the spout and which includes a puncture portion for liquid-tightly sealing the spout in the attached state and being capable of being punctured by the needle tip on a proximal end side of the needle tube, the support member supporting the double-ended needle movably in axial directions of the double-ended needle; and a cover member including a fixing portion fixed to the double-ended needle, the cover member covering the needle tip on a distal end side of the needle tube in a state where the fixing portion is fixed, and being operated to displace the double-ended needle along the axial directions of the double-ended needle. In an initial state, the needle tip on the distal end side of the needle tube of the double-ended needle is covered by the cover member and the needle tip on the proximal end side of the needle tube thereof is in a not-yet-punctured state with respect to the puncture portion. During a period of time from the initial state to a usable state where the cover member, along with the double-ended needle, is displaced in the proximal end direction, in the double-ended needle, the needle tip on the proximal end side of the needle tube punctures the puncture portion so that the needle tube communicates with the liquid injection device. In addition, fixation of the fixing portion to the double-ended needle is released so that it becomes possible for the cover member to be detached from the double-ended needle. With this constitution, the cover member covering the double-ended needle can surely be prevented from being involuntarily detached from the double-ended needle. In other words, the cover member can surely be prevented from being detached from the double-ended needle before the needle tube of the double-ended needle punctures the puncture portion of the support member attached to the spout of the liquid injection device and then the needle tube communicates with the liquid injection device. Thus, it is possible to surely prevent the liquid injection device from being involuntarily brought into the usable state. Therefore, a problem can be eliminated in that the liquid injection device is wastefully used or has to be discarded. Hence, the medical device of the present invention has industrial applicability.

## Claims

1. A medical device including a liquid injection device having at a distal end portion a spout through which liquid passes and a double-ended needle assembly attached to the spout of the liquid injection device,
the double-ended needle assembly comprising:
a double-ended needle including a needle tube having incisive needle tips at both ends, and a hub for supporting the needle tube;
a support member attached to the spout and which includes a puncture portion for liquid-tightly sealing the spout in the attached state and being capable of being punctured by the needle tip on a proximal end side of the needle tube, the support member supporting the double-ended needle movably in axial directions of the double-ended needle; and
a cover member including a fixing portion fixed to the double-ended needle, the cover member covering the needle tip on a distal end side of the needle tube in a state where the fixing portion is fixed, and being operated to displace the double-ended needle along the axial directions of the double-ended needle;
wherein in an initial state, the needle tip on the distal end side of the needle tube of the double-ended needle is covered by the cover member and the needle tip on the proximal end side of the needle tube thereof is in a not-yet-punctured state with respect to the puncture portion, and
during a period of time from the initial state to a usable state where the cover member, along with the double-ended needle, is displaced in the proximal end direction, in the double-ended needle, the needle tip on the proximal end side of the needle tube punctures the puncture portion so that the needle tube communicates with the liquid injection device, and fixation of the fixing portion to the double-ended needle is released so that it becomes possible for the cover member to be detached from the double-ended needle.

2. The medical device according to claim 1,
wherein the communication between the needle tube and the liquid injection device is done at the same timing as or at an earlier timing than the release of the fixation of the cover member to the double-ended needle.

3. The medical device according to claim 1,
wherein the hub is composed of a tubular body,
the cover member is composed of a tubular body disposed on the outer side of the hub, and has a fixing projection for functioning as the fixing portion, the fixing projection being formed on an inner circumferential portion of the cover member so as to project therefrom, and
the fixing projection engages with a side wall of the hub in the initial state and thus is fixed to the double-ended needle, and in the usable state the fixing projection is pushed outwardly by a portion of the liquid injection device and is separated from the side wall of the hub, whereby the engagement of the fixing projection with the side wall of the hub is released.

4. The medical device according to claim 3,
wherein the side wall of the cover member is composed of a plurality of small pieces that are supported at respective distal ends in a cantilever manner and disposed along a circumferential direction thereof, and connecting portions for connecting proximal end portions of the small pieces that are adjacent to each other, and the fixing projection is disposed on the connecting portion.

5. The medical device according to claim 1, further including positioning means for axially positioning the double-ended needle with respect to the support member in each of the initial state and the usable state.

6. The medical device according to claim 5,
wherein the hub is composed of a tubular body,
the support member is composed of a tubular body disposed on the inner side of the hub, and
the positioning means includes a positioning projection projecting from a side wall of one of the hub and the support member; a first engaging portion provided on a side wall of the other of the hub and the support member and which engages with the positioning projection in the initial state; and a second engaging portion provided closer to a proximal end side than the first engaging portion and which engages with the positioning projection in the usable state.
